(19) Europäisches Patentamt
European Patent Office
Office européen des brevets

(11) Publication number: **0 538 330 B1**

(12) **EUROPEAN PATENT SPECIFICATION**

(45) Date of publication of patent specification: **11.10.95**

(51) Int. Cl.$^6$: **C07C 323/25**, C12N 5/00, A61K 31/095, A61K 31/16

(21) Application number: **91912761.3**

(22) Date of filing: **08.07.91**

(86) International application number: **PCT/US91/04725**

(87) International publication number: **WO 92/00960 (23.01.92 92/03)**

(54) **BETA-ALETHINE USE IN CELL CULTURE AND THERAPY.**

(30) Priority: **06.07.90 US 549103**
**06.07.90 US 549104**

(43) Date of publication of application: **28.04.93 Bulletin 93/17**

(45) Publication of the grant of the patent: **11.10.95 Bulletin 95/41**

(84) Designated Contracting States: **AT BE CH DE DK ES FR GB GR IT LI LU NL SE**

(56) References cited:
**US-A- 4 552 765**

**PATENT ABSTRACTS OF JAPAN vol. 8, no. 178 (C-238)16 August 1984 & JP-59 073 515 ( SANTEN SEYAKU KK ) 25 April 1984**

**J.REPROD.FERTIL. vol. 57, 1979, pages 505 - 10**

**J.MED.CHEM. vol. 29, 1986, pages 2217 - 25**

**SCIENTIFIC AMERICAN vol. 254, no. 1, January 1986, pages 40 - 47**

(73) Proprietor: **UNIVERSITY OF NEW MEXICO**
**102 Scholes Hall**
**Albuquerque, NM 87131-6003 (US)**

(72) Inventor: **KNIGHT, Galen, Daryl**
**1116 Gold, SW**
**Albuquerque, NM 87102 (US)**
Inventor: **MANN, Paul, Leslie**
**3308 Loma Vista Pl., NE**
**Albuquerque, NM 87106 (US)**
Inventor: **SCALLEN, Terence, Joseph**
**7412 El Morro Rd., NE**
**Albuquerque, NM 87110 (US)**

(74) Representative: **Weatherald, Keith Baynes et al**
**15 Alders Road**
**Reigate,**
**Surrey RH2 0EA (GB)**

**EP 0 538 330 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

GOVERNMENT RIGHTS

This invention was made in the performance of work under grants #HL 16,796, #AM 10,628, and #SO7RR-05583-25 with the National Institutes of Health, and the United States Government has certain rights therein.

1. BACKGROUND OF THE INVENTION

The invention provides a compound for the inducement of cell differentiation, adaptation of cells to culture, and enhancement of cell phenotypic expression, vitality, longevity, and production. In particular, the invention provides a cell differentiation compound useful for inducing the differentiation of precursor cells into specialized cells, for normalizing function of malfunctioning cells, and for eliminating intractable cells (cells which are "resistant to cure, relief, or control", Dorland's Illustrated Medical Dictionary, 26th Edition, 1974, page 675, W. B. Saunders, Philadelphia).

Cellular differentiation is a well-known phenomenon which broadly refers to processes by which precursor cells (commonly termed "stem cells") develop into specialized cells. Differentiation compounds, i.e., compounds which induce differentiation of cells, have been clearly distinguished from growth factors i.e., those factors which induce cell multiplication, in the literature (see, e.g., "Growth, Differentiation, and the Reversal of Malignancy", Scientific American pp. 40-47, January, 1986, and the publications cited therein) and the implications of each with respect to therapeutic use in the treatment of disease or disorders of the body are of much current interest. The present application relates to the identification of $\beta$-alethine as a non-cell-lineage-dependent differentiation compound, and the use of $\beta$-alethine to induce differentiation and/or normalization of the function of a variety of cells, particularly for therapeutic benefits.

"Phenotypic cell expression" is defined herein as the manifestation of an entire range of physical, biochemical, and physiological characteristics of an individual cell as determined both genetically and environmentally, in contrast to "genotypic cell expression", which in the art solely refers to the expression of the cell chromosomal sequence. [See, for example, Dorland's Illustrated Medical Dictionary, 26th Edition, 1974, W. B. Saunders, Philadelphia]. Biological activity of the compounds of the invention thus includes modulation of the expression of genetic material of cells in culture as influenced by the condition and environment of each cell, including the age of the cell, the culture or conditions employed, and the presence of optionally added biological effectors.

2. DISCUSSION OF RELATED ART

$\beta$-alethine is known to be produced in vivo as a by-product of metabolic pathways. It is related via these pathways to pantothenic acid, which is a vitamin having known nutritional benefits (see, e.g., J.Reprod.Fert. 57: 505-510 (1979), and related compounds have been suggested for use in conjunction with radiotherapy as radioprotectors (J.Med.Chem. 29: 2217-2225, 1986: WO 85/00167, Jan. 17, 1985). No other relevant asserted biological functions of this compound are known to be described in the prior art. The compound is primarily well-known as a starting material for the chemical synthesis of related compounds (see, e.g., Japanese patent applications (83) 198461; (83) 46063A2; (81) 156256A2; (81) 104861A2; (80)124755; (75)-62932; (80) 07222; and U.S patents 2,835,704 and 4,552,765; for examples of the preparation of $\beta$-alethine, $\beta$-aletheine, pantetheine, and its derivatives or intermediates, and also for Coenzyme A and Coenzyme A derivatives or intermediates).

3. SUMMARY

The invention accordingly provides methods for inducing cell differentiation and normalization of cell function, and for enhancing cell phenotypic expression, vitality, longevity, and production employing $\beta$-alethine as differentiation compound. The invention has particular application in the differentiation of mammalian cells, including human cells, both in vitro and in vivo, most especially for normalizing cell development with respect to both cell maturation and differentiation-dependent cell growth; however, the use of $\beta$-alethine as differentiation compound for other cells, such as reptilian, avian, plant, insect, arachnid, rickettsial, bacterial, yeast, mold, protozoan, and fungus cells is also contemplated. Exemplary applications include the following: normalization of immunodeficient and autoimmune cell function (including the therapeutic use of $\beta$-alethine in the treatment of immunodeficiency and autoimmune diseases and disorders, particularly in mammals, and especially in humans); delay of cell senescence (including the therapeutic use of β-alethine in the treatment of diseases or disorders characterized by presenescent or prematurely senescing cells, particularly in mammals, and especially in humans); enhancement of cellular phenotypic expression, production, and vitality, and the therapeutic benefits derived therefrom; and adaptation of resistant cells to culture and the diagnostic and therapeutic uses derived therefrom. Within the scope of the present invention, β-aletheine, the reduced form of β-alethine, is to be considered the biological equivalent of β-alethine for purposes of practicing the invention, as β-alethine is readily reduced to β-aletheine, in vivo , for example by abundant intracellular thiol compounds and enzymes, such as glutathione and thiol-disulfide isomerases in mammals. Both compounds have the advantage of having inherent antioxidative properties; however, β-alethine is chemically more resistant to autoxidation than β-aletheine, and the use of β-alethine in the present invention is generally preferred for this reason.

## 4. BRIEF DESCRIPTION OF THE DRAWING

FIG. 1 illustrates data from a series of experiments designed to test the effect of β-alethine on the maximum population doubling level (PDL) of IMR-90 human fetal lung fibroblasts.

Fig. 2 illustrates data from a series of experiments designed to study the effect of β-alethine on non-antigen specific immunoglobulin synthesis and secretion by human peripheral blood leukocytes (HPBLs) in vitro.

Fig. 3 illustrates data from a series of experiments designed to study the effect of β-alethine on murine splenocyte production of non-specific immunoglobulin.

Fig. 4 illustrates data from an experiment designed to study the effect of β-alethine on the ability to adapt cells taken from in vivo to in vitro growth.

Figures 5-7 illustrate early and late tumor development in mice inoculated with non-secreting myeloma cells (NS-1) and treated with varying doses of β-alethine.;

Figure 8 is a three-dimensional composite of the data of Figures 5-7;

Figure 9 is an 85 degree clockwise rotation of the illustration of Figure 8; and

Figure 10 illustrates modulation of tumor in a mouse with differing amounts of β-alethine.

## 5. DETAILED DESCRIPTION OF THE INVENTION

As indicated above, β-alethine is a known compound [$(H_2NCH_2CH_2(C=O)NHCH_2CH_2S)_2$ and Figure 1, following] commonly produced by oxidation of the corresponding monosulfide, β-aletheine ($H_2NCH_2CH_2$-$(C=O)NHCH_2CH_2SH$ and Figure II, following), which is unstable in air and aqueous solutions [The Merck Index, 9th edition (#221), Merck & Co., Rahway, NJ]:

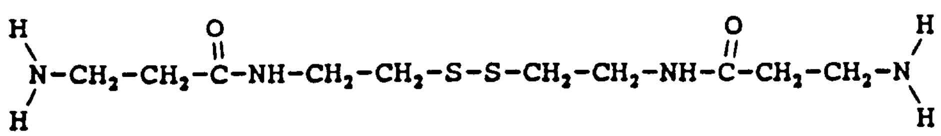

(I)

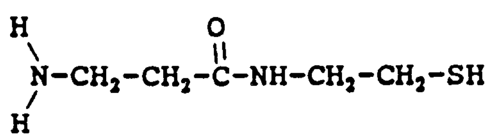

(II)

Both compounds are stabilized as their acid salts, particularly their hydrogen halide salts, and especially their hydrochloride salts. Various techniques for the synthesis of β-alethine based on deblocking of (N,N'-bis-carbobenzoxy)-blocked β-alethine are described in the literature (carbobenzoxy is often abbreviated as CBZ); however, most of the known procedures result in an unsatisfactory yield or purity of product, or both. Accordingly, it is preferred that β-alethine for use in the processes of the invention be prepared by processes which ensure purity of product and preferably also maximize yield, for example by the process of the invention comprising coupling N-CBZ-blocked β-alanine to N-hydroxysuccinimide to produce the corresponding active ester, which is then coupled to cystamine prepared by oxidation of cysteamine with hydrogen peroxide; the product, bis-CBZ-blocked β-alethine, is then recovered and deblocked. The process is described in detail in the Examples, and provides a high-yield, high-purity product suitable for

pharmaceutical use.

According to the invention, β-alethine appears to regulate a set of generic differentiation mechanisms that are not cell-lineage specific and that are common to cells regardless of phenotypic specialization. Consistent with this premise, the use of β-alethine according to the invention is not significantly dosage-dependent with respect to cell lineage, phenotype, or point of intervention in the cell cycle, except as noted below. Dosages starting from about 100 pg/ml culture are useful for cellular differentiation. For in vivo applications, amounts from about 10 pg/kg of body weight are recommended, particularly amounts from about 10 pg/kg up to about 200 μg/kg, and more particularly, up to about 100 μg/kg, which may be administered by any customary route in the presence of conventional carriers (such as physiological saline for non-oral routes including parenteral, or with suitable entero-coating in oral routes) preferably on a daily or alternate-daily regimen as described more fully below, until the desired results are achieved, although other regimens, such as weekly or biweekly regimens may suffice, particularly when results are apparent; i.e., decreases in dosages as normalization progresses may be suitable. Use of amounts of β-alethine substantially in excess of those required to obtain differentiation, normalization of cell function, or other results noted herein is not recommended, as excessive dosages may be counterproductive or at least ineffective. For in vitro applications, dosages starting from about 10 pg/ml culture are suggested, with replenishment as described below.

It is contemplated that β-alethine is useful for the differentiation of cells of living organisms in general, including mammalian (especially human), reptilian, avian, plant, insect, arachnid, rickettsial, bacterial, yeast, mold, protozoan, and fungus cells, owing to the commonality of results obtained with corresponding dosages observed in both experiments reported herein and unreported experiments and the ubiquitous presence of precursors to β-alethine in living systems. Further, β-alethine is useful for adapting to culture cells which are generally not regarded as so adaptable (herein referred to as culture-resistant cells), such as hepatic cells.

According to the invention, β-alethine comprises a differentiation compound which normalizes cell function (i.e., increases insufficient function, or decreases excess function); promotes cell longevity and/or bioproductivity; and/or diversifies cell function (i.e., expands phenotypic cellular expression). β-alethine specifically functions to (1) adapt resistant cells to culture; (2) delay senescence of cells in vitro, wherein senescence is broadly defined as the cell's increasing inability to reproduce itself in culture, typically characterized by markedly increasing cell generation times ($T_g$) at specific population doubling levels (PDL); or the time required for one complete round of cell division (see, e.g., Hayflick, R., Exp.Cell.Res. 37: 614-636 (1965), incorporated herein by reference, for a discussion of markers of senescence including $T_g$ and PDL); and (3) normalize or improve function, such as immunological surveillance (the recognition and elimination of intractable cells), and/or production of cells, especially those of the immune system (immunocytes). Particularly for delay of senescence in cell cultures, it is preferable to expose cells to be treated to β-alethine prior to significant cell malfunction (in this case, onset of senescence), as it has been found difficult, for example, to reverse cellular senescence once it has begun; thus treatment with β-alethine according to the invention includes prevention of cellular differentiative malfunction as described herein, as well as therapeutic treatment of existing differentiative malfunction, such as that associated with various diseases or disorders. Markers of abnormal cellular differentiative function denoting incipient diseases or disorders, including markers of approaching cellular senescence, are well-known or developing in the art, and practitioners are referred to the literature for methods for assessing such markers.

In order to normalize the life cycle of cells in culture, i.e., optimize growth and maturation of cells with respect to senescence and death, and adapt resistant cells to culture, it is preferred that the cells be exposed to β-alethine before the onset of senescence. Since cellular ageing is a gradual procedure, senescence may to some degree be arrested even if the cells are exposed to the compound at a later stage in the life of the cells, depending upon the particular cell type, culture conditions and other factors. However, senescent cells are less viable and productive by definition, so maintaining them at this late stage of the lifespan is counterproductive for most aspects of the invention, unless, for example, study of senescent cells is of concern. Consequently, for optimum results in most instances (e.g., when optimization of cell life and function is desired) it is preferable to expose cells to the compound as early in their life-cycle as is convenient.

Culture-resistant cells (i.e., cells which have a brief lifespan under conventional culture conditions, for example of two weeks or less; or those which do not express normal biofunctions in culture, such as those wherein normal production of hormones, enzymes, or other bioproducts is suppressed in vitro) are adaptable to culture by early exposure to adaptive amounts of the compound, preferably by combining the compound with the culture medium before introducing the cells. Exemplary resistant cells include lymphoid, hepatic, pancreatic, neural, thyroid, and thymus mammalian cells.

β-alethine may be added to any known culture medium, optionally supplemented with protein components such as serum, e.g., fetal or new-born calf serum, to obtain the results of the invention; the media employed do not form a part of this invention. Exemplary media include Eagle's Basal Medium; Eagle's Minimal Essential Medium; Dulbecco's Modified Eagle's Medium; Ham's Media, e.g., F10 Medium or F12 Medium; Puck's N15 Medium; Puck's N16 Medium; Waymoth's MB 5421 Medium; McCoy's 5A Medium; RPMI Media 1603, 1534, and 1640; Leibovitz's L15 Medium; ATCC (American Type Culture Collection) CRCM 30; MCDB Media 101, 102, 103, 104; CMRL Media 1066, 1415; and Hank's or Earl's Balanced Salt Solution. The basal medium employed, as known in the art, contains nutrients essential for supporting growth of the cell under culture, commonly including essential amino acids, fatty acids, and carbohydrates. The media typically include additional essential ingredients such as vitamins, cofactors, trace elements, and salts in assimilable quantities. Other factors which promote the growth and maintenance of cells, including compounds and factors necessary for the survival, function, production, and/or proliferation of the cells, such as hormones, for example peptidyl or steroidal hormones, growth factors, and antibiotics are also typically included. The media also generally include buffers, pH adjusters, pH indicators, and the like.

Media containing the modulators of the invention are applicable to a variety of cells, especially eukaryotic cells. The media of the invention are suitable for culturing animal, especially mammalian cells including human cells; plant cells; insect cells; microorganisms such as bacteria, fungi, molds, protozoa, and rickettsia, especially antibiotic-producing cells. β-alethine is broadly useful for promoting viability of living cells in a broad spectrum of so-called tissue culture media adapted for the culture of such cells. Exemplary applications include the culture of cloned cells, such as hybridoma cell lines; of cells, mammalian cells in particular and especially human cells, for the production of cell products, particularly proteins and peptides such as hormones, enzymes, and immunofactors; of virally-infected cells for the production of vaccines; of plant cells in, for example, meristem or callus culture; of epithelial cells to provide tissue for wound healing; of resistant cells for medical and diagnostic use; and in media adapted for the production and preservation of biological organs and implant tissue.

Specific cell types useful for culture in the processes of the invention accordingly include: cells derived from mammalian tissues, organs, and glands such as the brain, heart, lung, stomach, intestines, thyroid, adrenal, thymus, parathyroid, testes, liver, kidney, bladder, spleen, pancreas, gall bladder, ovaries, uterus, prostate, and skin; reproductive cells (sperm and ova); lymph nodes, bone, cartilage, and interstitial cells; blood cells including immunocytes, cytophages such as macrophages, lymphocytes, leukocytes, erythrocytes, and platelets. Additional cell types include stem, leaf, pollen, and ovarian cells of plants; microorganisms and viruses as described above; and cells derived from insect tissues, organs, and glands.

Culture techniques useful in conjunction with the modulators of the invention include the use of solid supports, (especially for anchorage-dependent cells in, for example, monolayer or suspension culture) such as glass, carbon, cellulose, hollow fiber membranes, suspendable particulate membranes, and solid substrate forms, such as agarose gels, wherein the compound is caged within the bead, trapped within the matrix, or covalently attached, i.e. as a mixed disulfide. β-alethine is useful in primary cultures; serial cultures; subcultures; preservation of cultures, such as frozen or dried cultures; and encapsulated cells; cultures also may be transferred from conventional media to media containing the compound by known transfer techniques.

According to the practice of this one aspect of the invention, cells are treated with β-alethine in an amount effective to promote culture of these cells in vitro, as measured, for example, by significant increase in cell viability or lifespan, increase in cell biomass, or increase in cell bioproductivity, as compared to untreated cells. For in vitro applications, from about 10 pg/ml of cell culture (based on a density of from about $10^5$ to about $10^7$ cells/ml) are suggested. The culture should be replenished with the compound as necessary, generally on a daily basis; again, treatment on an alternate-day or biweekly basis may suffice, depending upon the desired results.

In immunological applications, immunocytes are exposed to β-alethine to promote and\or diversify the function of immunocytes such as leukocytes, lymphocytes, splenocytes, T-cells, B-cells, natural killer (NK) cells, and cytophages such as macrophages. In particular, β-alethine is useful in vivo or in vitro to diversify or improve splenocyte or lymphocyte production of antibodies for use in monoclonal antibody systems; to diversify or improve immunoglobulin production; to generally promote normal immunofunction of immunocytes; and to treat diseases or disorders, especially those of the immune system, by treating the organism, mammals in particular and especially humans, with effective dosages of β-alethine (optionally including an agent that promotes the growth and maintenance of cells described above) either directly, or by removing the affected cells, treating them in vitro, and reinjecting them into the affected organism. Human diseases contemplated to respond to these therapies include autoimmune diseases, hypogammaglobulinemia, and AIDS (acquired immune deficiency syndrome).

## 6. EXAMPLES

### I. Preparation of β-alethine:

β-alethine was produced by deblocking N,N'-bis-carbobenzoxy (CBZ) blocked β-alethine produced as follows:

#### A. Preparation of N,N'-bis-(CBZ)-β-alethine or 5,5-Bis[N-carbobenzoxy-β-alanyl)-2-aminoethyl] disulfide.

A solution of dicyclohexylcarbodiimide (23.3g) was added to a solution of N-CBZ-β-alanine (24.84g) and N-hydroxy-succinimide (12.92g) in a total volume of about 500 ml of dry 10% acetonitrile in dichloromethane. Dicyclohexylurea (24.51g) precipitated as a by-product upon formation of the active ester. The active ester was dried to an oil and triturated with anhydrous ethyl ether. The precipitate was resuspended in dichloromethane and additional dicyclohexylurea was allowed to precipitate. The resulting dichloromethane solution of active ester was filtered and added to a previously prepared solution of cystamine (8.5g). The desired product, N,N'-bis-(CBZ)-β-alethine precipitated from this mixture. The mother liquor, anhydrous ether, dichloromethane extracts of the product, and the anhydrous ether extract of the active ester recovered above were dried and recombined to augment the yield of product. The product was substantially insoluble in water, hot (above about 70 °C) ethyl acetate, and hot (above about 30 °C) ether, and these can be used to further extract impurities. The product can also be recrystallized from dimethyl sulfoxide with acetonitrile or water, and again rinsed with ethyl acetate and ether. The latter process results in a 1 °C increase in product melting point, from 180 to 181 °C (uncorrected). Yields of N,N'-bis-(CBZ)-β-alethine of up to theoretical yields are contemplated; yields of 85-90% of theory have been routinely obtained. When dried over $P_2O_5$, *in vacuo*, the product appears to retain one mole equivalent of water, and was analyzed accordingly as the monohydrate.
Anal. Calcd. for $C_{26}H_{34}N_4O_6S_2H_2O$:C, 53.78; H, 6.25; N, 9.65. Found: C, 54.23; H, 6.56; N, 9.66. Sample analyzed by Ruby Ju, Department of Chemistry, University of New Mexico, Albuquerque, New Mexico.

#### B. Deblocking of CBZ-blocked β-alethine obtained from I.A., above [preparation of β-alethine•2HCl; or N,N'-bis-β-alanyl)-cystamine; or N,N'-bis-(β-alanyl-2-aminoethyl) disulfide].

Complete removal of the carbobenzoxy group was accomplished according to procedures described in J.Am.Chem.Soc. 86: 1202-1206 (1964), incorporated herein by reference. After deblocking with four equivalents of hydrogen bromide in glacial acetic acid per mole of the N,N'-bis-(CBZ)-1-alethine for 15 hours, the β-alethine was purified by precipitation with acetonitrile, rinsing with anhydrous ethyl ether, resuspension in water and filtering, and precipitating the mixed salt product with acetonitrile. Initial yields were in excess of 80% of theoretical maximum yields. β-alethine was converted to the hydrochloride salt by passing the preparation over a 30 ml X 15 cm long column of Dowex AG 1X8 (chloride form) (Dow Chemical Corp., Midland, Michigan) which had been previously prepared by eluting with 1M KCl and rinsing thoroughly with DI (deionized) water. Neutralization with $Ca(OH)_2$ and recrystallization of the β-alethine HCl from water with acetonitrile resulted in fine needles which melted at 224-225 °C (uncorrected).
Anal. Calcd. for $C_{10}H_{22}N_4O_2S_2$•2HCl: C, 32.69; H, 6.59; N, 15.25. Found: C, 32.52; H, 6.69; N, 15.32. Sample analyzed by Ruby Ju, Dept. of Chemistry, University of New Mexico, Albuquerque, NM.

C. Characterization of β-alethine: $[^{13}C]$-NMR; $[^{1}H]$-NMR; and IR spectra of β-alethine.

| | a | b | c | d | e | f |
|---|---|---|---|---|---|---|
| | S-$CH_2$ | $CH_2$-N | H-N-C=O | O-C-$CH_2$ | $CH_2$-N | -N-H<br>/ \<br>H + H |
| | | | $[^{13}C]$-NMR | | | |
| β-alethine | 37.59 | 39.04 | 172.79 | 32.9 | 36.71 | ----- |
| | | | $[^{1}H]$-NMR | | | |
| β-alethine | 2.524 | 3.094 | ----- | 2.694 | 3.367 | ----- |
| | | | | | | H-N-C=O<br>\<br>O-R |
| bis-(CBZ)-<br>-β-alethine<br>(DMSO) | 2.740 | 3.309 | 8.085 | 2.254 | 3.192 | 7.24 |
| | a | b | c | d | e | f |
| | | | IR ($cm^{-1}$) | | | |
| | a | b | c | d | e | f |
| | | | | | | -N-H<br>/ \<br>H + H |
| β-alethine | 660w | | 3250w<br>1555w-s<br>1286m<br>1620s | ---- | | 3270v<br>2970s-w<br>1462s<br>1620s<br>1128s |
| | | | | | | H-N-C=O<br>\<br>O-R |
| bis-(CBZ)-<br>-β-alethine | ---- | | 3345s<br>1545m<br>1640s | ---- | | 3345s<br>1535s<br>1270m<br>1682s |
| | a | b | c | d | e | f |

(R is a benzyl moiety in this table.)

β-alethine is unusual in that changes in pH [neutralization with $Ca(OH)_2$] cause pronounced shifts in the positions and intensities of IR bands.

Peaks (HCl salt): 3270s, 3170s, 2970s, 2700w, 2550w, 2020w, 1657s, 1595m, 1560s, 1450s, 1409m, 1390w, 1354w, 1325m, 1300w, shoulder/1252m/shoulder, 1188m, 1129m, 1097m, 1079w, 1030w, 950w, 905w, 829m.

Peaks (neutralized): 3250w, 3180w, 2940m/broad, 2375s, 2230s, 2157s, 1936w, 1620s, 1555w, 1462s, 1432 shoulder, 1400m, 1342m, 1286m, 1217m, 1188m, 1128s, 1020m, 810w, 719m, 660w.

Bis-(CBZ)-β-alethine displays only a few of the resonances present in β-alethine.

Peaks: 3345s, 3310s, 1682s, 1640s, 1545m shoulder, 1535s, 1450w, 1427w, 1375w, 1332m, 1270m, 1231m, 1178w, 1120w, 1030m/broad.

II. Delay of Cellular Senescence of Fibroblasts with β-alethine.

Figure 1 shows data from a series of experiments designed to test the effect of β-alethine on the maximum population doubling level (PDL) of IMR-90 human fetal lung fibroblasts. This cell line is available from the American Type Culture Collection (ATCC, Bethesda, MD, USA) and is used as a standard for in vitro cellular senescence studies. Cellular senescence is loosely defined as those cellular process(es) that together result in the cell's inability to replenish itself in culture. This model represents ageing processes in vivo, and cell lines developed from humans of different ages have PDL's in vitro which are inversely related to the chronological age of the donor. The IMR-90 cell line has been documented as having a maximum PDL under ideal tissue culture conditions of approximately 45 PDLs. The IMR-90 cells depicted in Figure 1 were grown under ideal conditions in McCoy's 5A synthetic medium supplemented with HEPES buffer at 10 mM; 100 units penicillin G/ml and 100 µg streptomycin/ml at standard concentrations; new born calf serum (NBCS) at 20% (V/V); and L-glutamine at 2 mM. The treated cell cultures were augmented with different concentrations of β-alethine (as indicated) dissolved in phosphate-buffered saline (PBS)---always in a standard volume of 30 microliters per culture. The β-alethine was added to the cultures at PDL 35 which is considered Phase II or midlife of these cells in chronological terms, at the time the cells were passaged. The culture process comprised removing the adherent cells from their substrate by treating the cells with 0.25% trypsin/EDTA (ethylenediamine tetraacetic acid) solution for two to five minutes. The cells were then washed twice with the complete medium and counted in a double Neubauer hemacytometer; 2 million cells were aliquoted into a fresh tissue culture flask (T-75, polystyrene by LUX, Flow General, McClean, MD, USA) with 20 ml of fresh complete medium. This process was repeated every 48 to 72 hours when the cells reached approximately 80% surface confluency. Thus the cells were maintained under conditions which facilitate logarithmic growth, i.e., between 30 and 80% surface confluency. Under these conditions IMR-90 cells senesce at approximately PDL 45-47 (first bar on graph). Cells treated with β-alethine continued to grow well beyond this point and finally senesced in a dose-dependent manner from PDL 67 to PDL 101.5. During the β-alethine-dependent growth extension the cells were observed to have phenotypes similar to Phase II fibroblasts. At the point of eventual senescence their phenotype was similar to that of the PDL 47 untreated control. The augmentation of growth represented a doubling of the life expectancy of the cells and in absolute terms represented an increase in cell number (biomass) by a factor of 2 raised to the power 55, or 3.6 X $10^{16}$ fold. It was concluded that this was a differentiative phenomenon, based in part on the observation that the treated cells have similar generation times (the time required for one complete round of division) before and after treatment with β-alethine, beyond the normal senescence point.

III. Differentiation of a peripheral lymphoid organ with β-alethine.

Figure 2 illustrates data from a series of experiments designed to assess the effect of β-alethine on non-antigen-specific immunoglobulin synthesis and secretion in vitro by human peripheral blood leukocytes (HPBLs), generally characterized as a peripheral lymphoid organ primarily populated by medium-sized, mature lymphocytes. In these experiments, blood was taken from healthy male humans; the blood was then defibrinated on glass beads, and the leukocytes separated by centrifugation (buffy coat technique). Residual red blood cells were lysed with a brief treatment with 0.85% ammonium chloride. The leukocytes were counted and dispensed into 24 well tissue culture trays (LUX, Flow General, McClean, MD, USA) in 1 ml of RPMI 1640 basal medium supplemented with penicillin, streptomycin, 10% fetal calf serum, and L-glutamine. β-Alethine was added to test cultures at various concentrations in 30 microliter doses. The cells were harvested as indicated in Figure 2 at various times between 72 and 144 hours of culture and tested for antibody production using a conventional protein-A facilitated plaque assay (A-PFC or Ig-PFC). In this assay, protein-A was covalently conjugated to washed sheep red blood cells (SRBC's) using chromium chloride in saline (6 mg/100ml) and used as target in the plaque assay. In addition, aliquots of cells were also tested for proliferation status by treating them with 0.5 µCi of tritiated thymidine (6-9 Ci/mole) followed by assessing the level of incorporation of radioactivity into newly synthesized DNA. Figure 2 shows that β-alethine stimulated the HPBLs to produce immunoglobulin in a dose-dependent manner at approximately 60 times the untreated control levels. The optimal concentration was about 5 nanograms/ml culture. The proliferation index indicated a low level of increased thymidine incorporation at 5 ng/ml doses. This 2-to-3 fold increase over background has minor significance as compared to truly proliferative stimulants such as LPS (lipopolysaccharide) or PHA (phytohemagglutinin), which under similar conditions result in the incorporation of about 200,000 cpm of radioactivity, approximately 100 -200-fold that of control levels. It was concluded that the level of proliferation observed in the experiment was attributable to differentiation-dependent proliferation rather than to independent proliferative processes stimulated by β-alethine, or to an

increase in both the survival of cells and the retentive capacity for deoxyribonucleic acids associated with viable cells.

IV. Differentiation of a central lymphoid organ with β-alethine.

Figure 3 shows data from a series of experiments designed to study the effect of β-alethine on murine splenocyte production of non-specific immunoglobulin. The assays and culture techniques were the same as those described above (Example III) for the HPBL model. The animals, 4 to 6 week old female BALBc/J mice, were sacrificed by cervical dislocation, and their spleens aseptically removed and pressed through 90 mesh stainless steel screens. After several washes with the complete medium the cells were counted and dispensed into the 24 well trays and test cultures were dosed with the β-alethine. These cultures were also harvested over various culture times of from 72 to 144 hours of culture, and tested for antibody production and proliferation, as shown in Figure 3. Figure 3 illustrates that β-alethine markedly stimulated the murine splenocytes to produce immunoglobulin in a dose-dependent manner, with an illustrated optimum at approximately 10 ng/ml dosages. In this case there was no significant stimulation of proliferation, based on the thymidine assay described in Example III.

V. Adaptation of Culture-Resistant Cells (Hepatocytes) to Culture with β-alethine.

Figure 4 illustrates data from a single experiment designed to study the use of β-alethine for adapting cells to culture. The culture-resistant cells employed (hepatocytes) were taken from in vivo to in vitro growth. Murine hepatocytes were chosen owing to their being especially difficult to adapt to culture. It has been suggested that difficulty in adapting cells to culture is related to the relative degree of differentiation of the selected cells, i.e., the more highly the cells are differentiated, the more difficult it is to culture them; accordingly, the hepatocyte mixed population of cell types were selected for this experiment on the basis that these cells are highly differentiated. In this experiment a single liver lobe from one of the BALBc/J mice was excised, pressed through a 90 mesh stainless steel screen, washed, and placed in T-25 tissue culture flasks (10 for LUX, Flow General, McClean, MD, USA). All cultures were maintained in 10 ml of the same medium as described in Example III (RPMI-1640 plus fetal calf serum plus pen/strep plus L-glutamine) and the test cultures exposed to various concentrations of β-alethine as indicated in Figure 4. Figure 4 shows that no colonies of hepatocytes were found in the control culture, while approximately 50 colonies were observed in cultures treated with 10 ng/ml of β-alethine. It was possible to obtain some viable colonies from the control cultures, but only if 10- to 20- fold higher initial cell concentrations were used; therefore the β-alethine was between 500 and 1000-fold more efficient in adapting murine hepatocytes to in vitro culture than control, i.e., saline-treated medium. In addition, the β-alethine-treated cultures were stable in culture, in contrast to the control cultures which are notoriously unstable in long-term culture. The effect is again dose-dependent, as illustrated.

VI. Treatment of Neoplasia with β-alethine

The invention further provides the use of β-alethine for The preparation of a medicament for treating neoplasias. In particular, this compound can be used for reducing tumor burden, inhibiting tumor growth, and inhibiting tumor intravascularization, for example from metastasizing tumors. β-alethine has been identified as a compound inducing cell differentiation and modulating cell growth, phenotypic expression (including bioproduction and function), vitality, and longevity, supra, and in copending U. S. application SN 07/549,104 entitled "Beta-Alethine Use in Cell Culture and Therapy"; a correspondence between cell differentiation and reversal of malignancy has been suggested; see, for example, "Growth, Differentiation, and the Reversal of Malignancy", Scientific American pp. 40-47, January, 1986, and the publications cited therein.

The present application relates to the further identification of β-alethine as a non-cell-lineage-dependent anti-tumor compound, and the use of β-alethine to induce normalization of the function of a variety of neoplastic cells, particularly for therapeutic benefits. The invention accordingly further provides the use of β-alethine for the recognition, normalization, and elimination of neoplastic cells particularly for the treatment of cancer.

According to the invention, β-alethine appears to regulate a set of generic differentiation mechanisms that are not cell-lineage specific and that are common to cells regardless of phenotypic specialization. Consistent with this premise, the use of β-alethine according to the invention as an anti-tumor agent is not significantly dosage-dependent with respect to cell lineage, phenotype, or point of intervention in the cell

cycle, except as noted below. For in vivo applications, from about 10 pg of β-alethine/kg of body weight are recommended, particularly amounts from about 10 pg/kg up to about 200 µg/kg, and more particularly, up to about 100 µg/kg, which may be administered by any customary route including parenterally (such as i.p.) or anally in conjunction with conventional carriers such as physiological saline for non-oral routes, or orally with appropriate entero-coating. The compounds are preferably administered on a daily or alternate-day regimen as described more fully below, until the desired results are achieved, although other regimens, such as weekly or biweekly regimens may suffice, particularly when results are apparent; decreases in dosages as normalization progresses or tumor burden is reduced may be suitable, and may be preferable to avoid too-rapid regression of tumors accompanied by excessive physiological stress on the organism. Use of amounts of β-alethine substantially in excess of those required to obtain differentiation, normalization of cell function, decrease of tumor burden, or other results noted herein is not recommended, as excessive dosages may be counterproductive or at least ineffective. For in vitro applications for normalization of neoplastic cells, dosages starting from about 10 pg/ml culture are suggested, with daily or bi-daily replenishment.

It is contemplated that β-alethine is useful for the treatment of neoplasias of cells of living organisms in general, including mammalian, especially human, reptilian, avian, and plant cells, owing to the commonality of results obtained with corresponding dosages observed in experiments reported herein and unreported experiments. According to the invention, β-alethine comprises an anti-tumor compound which normalizes cell function (i.e., increases insufficient function or decreases excess function). β-alethine specifically functions to (1) inhibit tumor growth, especially that of malignant tumors; (2) regress tumors, especially malignant tumors; (3) inhibit tumor metastasis; and (4) normalize growth characteristics of neoplastic cells; and/or (5) improve recognition and/or elimination of neoplastic cells.

In cancer applications, neoplastic cells or immunocytes, or both neoplastic cells and immunocytes, are exposed to β-alethine to promote differentiation of the cells and normalize the cell cycle. Treatment of tumor cells is effectively segregated from treatment of the immunocytes by removing immunocytes from the afflicted mammal, including humans. The immunocytes are then treated in culture with β-alethine, or with a combination of β-alethine and tumor cells derived from the afflicted mammal, until either the immunocytes are activated, or the tumor cells are completely attenuated for health reasons, respectively. The activated immunocytes preferably devoid of metastatic tumor cells are then reinjected into the mammal. β-alethine is useful in vivo for reducing soft (hematolymphoid) tumor burden, particularly in mammals, especially in humans, and inhibiting intravascularization of tumor cells, especially cells of metastasizing tumors. The compounds are thus useful for reducing tumor burden, by inhibiting tumor growth or by inhibiting tumor metastasis, or both. In particular, β-alethine is contemplated to be useful in the treatment of numerous soft and lymphoid malignant tumors, such as lymphomas; leukemias; hepatocellular tumors; liver tumors; and Hodgkin's disease; especially tumors such as myelomas. β-alethine is contemplated inter alia as useful in the treatment of neoplasia 1) prophylactically; 2) as a primary therapy for inhibiting tumor growth, particularly that of slowly-growing tumors; and 3) as a supplemental therapy pursuant to surgical intervention for removal or debulking of tumors, particularly virulent or primary tumors. Treatment with β-alethine has been found to regress tumors, reduce tumor mass, inhibit tumor growth, inhibit tumor metastasis, and inhibit tumor ascites production.

It is recommended that anti-tumor therapy commence at the earliest tumor stage possible, particularly to avoid peripheral physiological complications caused by the presence or metastasis of large tumors. β-alethine for tumor therapy is administered by any convenient route as noted above, for example i.v. or i.p., in a suitable conventional carrier such as physiological saline at at least therapeutic threshold amounts; from about 1 ng/kg body weight up to about 100 µg/kg are particularly suitable, depending upon the stage of the tumor. Dosages toward the higher end of the therapeutic range are recommended for Stage II tumors and above, whereas dosages toward the lover end of the range are suitable for stage I or incipient tumors. For cancer prophylaxis, dosages ranging from about 10 pg/kg body weight, preferably from about 1 ng/kg up to about 100 µg/kg body weight are contemplated. Therapeutic regimens of alternate days for the dosages noted above for cancer treatment are suitable, and appear to be preferable, based on the observation that in vivo induction of biochemicals such as enzymes thought to be responsive to β-alethine therapy appears to follow chemical stimulation by about 48 hrs. Prophylactic regimens may be followed on a daily basis.

At least at the dosage levels indicated, β-alethine appears to be a substantially non-toxic compound in healthy mice, with no observed adverse side-effect.

A. Inoculation of mice with NS-1 myeloma cells.

NS-1 myeloma cells (ATCC TIB 18, P3/NS1/1-Ag4-1) were employed as inoculant; these cells have proven to be about 90% effective in establishing myelomas in mice according to the exemplified procedure, and the untreated myelomas are substantially fatal within about two weeks.

The cells were grown for several passages (preferably one week) in a sterile environment consisting of RPMI 1640 (Whittaker M.A. Bioproducts, Walkersville, MD, USA) containing 10% fetal calf serum (Hyclone Laboratories, Logan, UT, USA), 2mM L-glutamine, 5,000 units of penicillin, and 5 mg streptomycin in 75 $cm^2$ polystyrene tissue-culture flasks (Corning Glassworks, Corning, NY, USA) in a humidified chamber at 37°C and under 6% $CO_2$. To assure NS-1 propagation in vivo it is essential to remove DMSO (the cryostatic agent dimethyl sulfoxide) through several medium changes and dilutions; this also serves to maintain the cells in log-phase growth. Female BALBc/J mice were injected i.p. with $10^4$ cells in 0.1 ml of standard phosphate-buffered saline as soon as possible after weaning, transport, and indexing, as it has been found that the NS-1 cell line employed does not generally perform optimally in animals which are mature or which have equilibrated with their environment. The mice were maintained with Wayne Rodent Blox (Wayne Research Animal Diets, Chicago, IL, USA) ad lib. and tap water.

B. Treatment of inoculated mice (Supra) with β- alethine (early intervention).

1. Concentrations of β-alethine as obtained above (Example I) of 1 ng/kg, 1 pg/kg, 10 μg/kg, and 100 μg/kg (based on the body weight of the inoculated mice) were injected i.p. in 0.1 ml physiological saline starting the second day after tumor inoculation (day 2), and continuing every Monday, Wednesday, and Friday through day 47. This regimen was predicated on the observation that enzymes thought responsive to these compounds and which may play a role in the reported results are induced 48 hours after chemical stimulation. The inoculated mice were compared to a) untreated controls and b) to carrier-injected (saline-injected) controls.

2. Conclusions

β-alethine is effective for preventing the onset of NS-1 myeloma in BALBc/J mice over the concentration range from 10 pg β-alethine/kg mouse to 100 μg/kg mouse. Without treatment, 75% of the mice in the experiment either had to be euthanized or died as the result of tumor development. At doses of β-alethine below the effective threshold [i.e., below about 10 pg/kg, or at about 10 pg/kg or 1 ng/kg (data not illustrated for the latter)] one-third to two-thirds of the animals ultimately contracted tumor. At dosages approaching the maximal effective dose (i.e., above about 10 μg/kg, or at about 10 μg/kg or 100 μg/kg), only one mouse developed a palpable tumor, which persisted for 20 days but eventually regressed. Figures 5-7 illustrate early and late tumor development (based on weight of mouse not attributable to normal weight gain) in mice treated with decreasing concentrations of β-alethine (100μg, 10μg, and 10 pg per kg mouse, respectively). In Figure 7, the biphasic curve in the center illustrates early and late tumor development in these mice and corresponds to two deaths at this dosage (10 pg/kg mouse) of β-alethine which is the therapeutic minimal threshold for this compound in this model (Figure 10). Normal weight gain of the mice is slightly inhibited at 100 μg/kg (Figure 5), but not significantly at 10 μg/kg (Figure 6). At 10 μg/kg (Figure 6) one mouse developed a palpable tumor which persisted for 20 days but eventually regressed. At effective dosages of the antineoplastic compound β-alethine (from about 10 pg/kg body weight to about 10 μg/kg body weight), there is a striking difference between the weights of the mice (reflecting tumor burden) in the untreated control group (the vehicle-injected control group), compared with the mice in the treatment group.

The effectiveness of β-alethine in the treatment of NS-1 myeloma is further illustrated in Figure 8, comprising a three-dimensional representation of the study; and in Figure 9, comprising a 85° clockwise rotation of the illustration of Figure 8, with higher doses of the drug in the front to no doses of the drug in the back. The controls inoculated with tumor (far left, Figure 8) gain tumor and therefore weight (Z-axis) at an accelerated rate with respect to the day of the study (Y-axis). Mice receiving high doses of β-alethine (far right) develop at a near-normal rate and show no signs of chronic tumor. Ridges or increases in elevation illustrate tumor development at the lower concentrations (farther left on the X-axis) which are coded for the morphology of the developing tumor (Ta = ascites and Ts = solid tumors) and for deaths resulting from either ascites or solid tumors (Da or Ds respectively). Complete regression is indicated by R at the point at which the tumor is no longer palpable. When normalized to the initial weights of the mice in the control group and plotted (analyzed as in Figures 5-9), control mice (physiological saline injections only) displayed normal growth and development approximating the growth and development of the mice receiving tumor and 10μg β-alethine/kg mouse (Figures 6 and 8). This is

further illustrated in Figure 10 in a mouse in which a tumor is modulated with different concentrations of β-alethine (below). The only departure from the normal growth curve coincides with the appearance and disappearance of a palpable tumor.

A tumor appeared and regressed without any signs of malaise in a mouse undergoing a therapy of 10 µg/kg β-alethine (Figure 6). Tumors developing in untreated controls with some exceptions typically promoted ascites development, while tumors developing in mice undergoing therapy with β-alethine were with few exceptions solid masses; since it thus appears that β-alethine promotes consolidation of tumor into discrete masses, β-alethine is projected to be valuable in the design of treatment regimens involving surgical debulking.

C. Treatment of inoculated mice (Supra) with β- alethine (late intervention).

A single mouse developing a tumor late in the experiment (at 4 on the X-axis, Figure 8, approximately 40 days after the 10 pg β-alethine/kg mouse treatment was discontinued) was treated with 100 µg β-alethine/kg mouse to determine the effect of late therapeutic intervention on the treatment of the myeloma (Figure 10). Massive log-phase growth of tumor persisted along the right side of the mouse from shoulder to hip and in the abdomen for 10 to 14 days after treatment with the higher dose was begun, indicating pronounced infiltration of the tumor into extra-peritoneal tissues, and a considerable lag phase before the treatment became effective. The growth then ceased, and was followed by rapid reduction of both the tumor mass and the weight of the mouse. There was a brief period in which the malaise subsided and the mouse's rough coat improved. This coincided with a temporary stabilization (approximately 1 week) of the mouse's weight suggesting that the tumor also stabilized during this time. This was followed by another precipitous drop in the mouse's weight and obvious decrease in the tumor masses. The mouse was euthanized when the weight returned to normal even though a palpable mass remained in the abdomen. At the time the mouse was euthanized, phlebitis was evident in the extremities, possibly resulting from the processing of the tumor equivalent of roughly 10% of the body weight per day. After correcting for necrosis evident histologically, it was estimated that between 85 and 90% of the original tumor was either necrotic or resorbed at the time of euthanasia. Considering the rate of resorption, there would have been complete regression of the tumor if the therapy had been maintained for the full month.

Wasting of the tumor and not the mouse proper was confirmed by weighing the debulked carcass. In this mouse, unlike untreated controls, there was no gross evidence of infiltration of organs by the tumor, and the remaining tumor appeared necrotic (yellowish-green and granular like an old sponge). Histological examination of the tissues indicated remaining tumor cells in the skeletal muscle adjacent to the abdominal wall , the subcutaneous tissue, and a mammary gland. Hepatic, urogenital, and gastrointestinal tumors, as well as a variety of other tumors, have been consistently observed in untreated mice, indicating the highly invasive and metastatic nature of the NS-1 cell line; however, in the treated mouse, none of the remaining organs contained tumor cells. An apparent pathological bone fracture was observed in this mouse, but no tumor cells were evident in the marrow of this bone. It was thus tentatively determined that the bone demineralized due to rapid growth of the tumor, a process requiring calcium and phosphate, and that subsequent rapid extraskeletal deposition of calcium phosphate as the tumor was resorbed resulted in the fracture.

Based on this and other studies, it is recommended that in the presence of aggressive neoplasia (e.g., Stage II or above tumors), at least the primary tumor should be debulked surgically in conjunction with β-alethine therapy, preferably before or very soon after β-alethine therapy is commenced. It is further recommended that in some instances, β-alethine initial dosages of about 100 µg/kg used in late intervention therapy be gradually reduced to slow the resorptive process and permit the organism to adjust to the therapy. Gradual decreases of dosages (on an alternating 48 hour regimen) from about 100 µg/kg down to about 1 ng/kg are suggested as the tumor responds to the therapy.

## Claims

**1.** Use of β-alethine for the manufacture of a medicament for regulating the differentiation of immunocytes, neoplastic cells, presenescent cells, hybridoma cells or infected cells, comprising the step or exposing cells, *in vitro* or *in vivo*, to an amount of β-alethine, or a physiologically-compatible salt thereof, in an amount sufficient to normalise or improve function or bioproduction of the cells.

**2.** Use of β-alethine as claimed in claim 1, in which the treated cells are mammalian.

**3.** Use of β-alethine as claimed in claim 2, in which the cells had previously been infected with a pathogen, including a virus.

**4.** Use of β-alethine as claimed in any preceding claim, in which the cells are immunocytes exposed to sufficient β-alethine to normalise or improve immunological surveillance.

**5.** Use of β-alethine as claimed in any preceding claim, in which the cells are exposed *in vitro* at least 10 pg β-alethine/ml of cell culture or, *in vivo*, to at least 10 pg β-alethine/kg weight of the organism.

**6.** Use of β-alethine for the manufacture of a medicament for treating neoplasia in a mammal, comprising the step of administering to the mammal β-alethine, or a physiologically-compatible salt thereof, in an amount sufficient to reduce tumor burden, induce regression of the tumor, or inhibit tumor growth or metastasis, optionally in conjunction with debulking of a tumor.

**7.** Use of β-alethine as claimed in claim 6, in which the neoplasia is hematolymphoid.

**8.** Use of β-alethine for the manufacture of a medicament for treating immune disease or disorder in a mammal, comprising the step of administering to the mammal β-alethine, or a physiologically-compatible salt thereof, in an amount sufficient to improve or normalise immunological function or bioproduction in the mammal.

**9.** Use of β-alethine as claimed in claim 8, in which the disease or disorder is an auto-immune disease or disorder, or an immunodeficiency disease or disorder, including AIDS or hypogasmmaglobulinemia.

**10.** Use of β-alethine as claimed in claim 9, comprising the steps of extracting an immunocyte from the mammal, exposing the immunocyte *in vitro* to sufficient β-alethine to stimulate immune function or bioproduction thereof, and thereafter reintroducing the stimulated immunocyte into the afflicted mammal.

**11.** Use of β-alethine as claimed in claim 10, in which the mammal is afflicted with neoplasia, and the immunocyte is additionally stimulated by exposure to the neoplastic cell prior to the reintroduction of the immunocyte into the mammal.

**12.** Use of β-alethine for the manufacture of a medicament for delaying the onset of senescence, comprising exposing a presenescent cell, *in vivo* or *in vitro*, to β-alethine in an amount sufficient to delay senescence thereof.

**13.** Use of β-alethine for the manufacture of a medicament for adapting a culture-resistant cell of an organism to culture, comprising the step of exposing the resistant cell, *in vivo* or *in vitro*, to β-alethine in an amount sufficient to adapt the cell to culture.

**Patentansprüche**

**1.** Verwendung von β-Alethin zur Herstellung eines Arzneimittels zur Regulierung der Differenzierung von Immunozyten, neoplastischen Zellen, Preseneszenz-Zellen, Hybridomazellen oder infizierten Zellen, umfassend die Stufe in vitro oder in vivo der Exposition der Zellen, in einer ausreichenden Menge an β-Alethin oder dessen physiologisch kompatiblen Salzes, um die Funktion oder Bioproduktion der Zellen zu normalisieren oder zu verbessern.

**2.** Verwendung von β-Alethin nach Anspruch 1, wobei die behandelten Zellen Säugetierzellen sind.

**3.** Verwendung von β-Alethin nach Anspruch 2, wobei die Zellen zuvor mit einem Pathogen einschließlich eines Virus infiziert worden sind.

**4.** Verwendung von β-Alethin nach einem der vorhergehenden Ansprüche, wobei die Zellen Immunozyten sind, die ausreichend β-Alethin exponiert worden sind, um die immunologische Überwachung zu normalisieren oder zu verbessern.

5. Verwendung von β-Alethin nach einem der vorhergehenden Ansprüche, wobei die Zellen in vitro zumindest 10 pg β-Aletin pro ml Zellkultur oder in vivo zumindest 10 pg β-Alethin pro kg Gewicht des Organismus exponiert werden.

6. Verwendung von β-Alethin zur Herstellung eines Arzneimittels zur Behandlung von Neoplasie bei Säugetieren, umfassend die Stufe der Verabreichung von β-Alethin oder dessen physiologisch kompatiblen Salzes an ein Säugetier in einer ausreichenden Menge, um die Tumorbelastung zu reduzieren, die Regression des Tumors zu induzieren oder das Tumorwachstum oder die Metastasenbildung zu inhibieren, gegebenenfalls in Verbindung mit Verkleinerung oder Verringerung eines Tumors.

7. Verwendung von β-Alethin nach Anspruch 6, wobei die Neoplasie ein Haematolymphoid ist.

8. Verwendung von β-Alethin zur Herstellung eines Arzneimittels zur Behandlung von Immunerkrankungen oder -störungen bei einem Säugetier, umfassend die Stufe der Verabreichung von β-Alethin oder dessen physiologisch kompatiblen Salzes an ein Säugetier in einer ausreichenden Menge, um die immunologische Funktion oder Bioproduktion bei dem Säugetier zu normalisieren oder zu verbessern.

9. Verwendung von β-Alethin nach Anspruch 8, wobei die Erkrankung oder Störung eine Autoimmunerkrankung oder -störung oder eine Immunschwächeerkrankung einschl. AIDS oder Hypogammaglobulinämie ist.

10. Verwendung von β-Alethin nach Anspruch 9, umfassend die Stufen des Extrahierens eines Immunozyten von einem Säugetier, in vitro Exposition des Immunozyten in ausreichender Menge β-Alethin, um deren Immunfunktion oder Bioproduktion zu stimulieren und danach Wiedereinführen der stimulierten Immunozyten in das unter der Erkrankung leidende Säugetier.

11. Verwendung von β-Alethin nach Anspruch 10, wobei das Säugetier unter Neoplasie leidet und der Immunozyt zusätzlich durch Exposition der neoplastischen Zelle vor der Wiedereinführung des Immunozyten in das Säugetier stimuliert wird.

12. Verwendung von β-Alethin zur Herstellung eines Arzneimittels zur Verzögerung des Beginns der Alterung, umfassend die in vivo oder in vitro Exposition einer preseneszenten Zelle an β-Alethin in einer ausreichenden Menge, um deren Seneszenz zu verzögern.

13. Verwendung von β-Alethin zur Herstellung eines Arzneimittels zum Anpassen einer kulturresistenten Zelle eines Organismus an die Kultur, umfassend die Stufe der in vivo oder in vitro Exposition der resistenten Zelle an β-Aletin in einer ausreichenden Menge, um die Zelle an die Kultur anzupassen.

**Revendications**

1. Utilisation de β-aléthine pour la fabrication d'un médicament pour réguler la différentiation des immunocytes, des cellules néoplasiques, des cellules présénescentes, des cellules d'hybridome ou de cellules infectées, comprenant l'étape d'exposition des cellules, *in vitro* ou *in vivo*, à une quantité de β-aléthine, ou d'un sel physiologiquement compatible de celle-ci, en une quantité suffisante pour normaliser ou améliorer la fonction ou la bioproduction des cellules.

2. Utilisation de β-aléthine selon la revendication 1, dans laquelle les cellules traitées sont des cellules de mammifères.

3. Utilisation de β-aléthine selon la revendication 2, dans laquelle les cellules ont été auparavant infectées avec un agent pathogène, y compris un virus.

4. Utilisation de β-aléthine selon l'une quelconque des revendications précédentes, dans laquelle les cellules sont des immunocytes exposés à suffisamment de β-aléthine pour normaliser ou améliorer la surveillance immunologique.

5. Utilisation de β-aléthine selon l'une quelconque des revendications précédentes, dans laquelle les cellules sont exposées *in vitro* à au moins 10 pg de β-aléthine/ml de culture cellulaire ou, *in vivo*, à au

moins 10 pg de β-aléthine/kg de poids de l'organisme.

6. Utilisation de β-aléthine pour la fabrication d'un médicament pour traiter une néoplasie chez un mammifère, comprenant l'étape d'administration de β-aléthine, ou d'un sel physiologiquement compatible de celle-ci, au mammifère, en une quantité suffisante pour réduire la charge tumorale, provoquer la régression de la tumeur ou inhiber le développement ou la métastase de la tumeur, éventuellement conjointement avec la disparition de la tumeur.

7. Utilisation de β-aléthine selon la revendication 6, dans laquelle la néoplasie est hématolymphoïde.

8. Utilisation de β-aléthine pour la fabrication d'un médicament pour traiter une maladie ou un trouble immunitaires chez un mammifère, comprenant l'étape d'administration de β-aléthine, ou d'un sel physiologiquement compatible de celle-ci, au mammifère, en une quantité suffisante pour améliorer ou normaliser la fonction ou la bioproduction immunologiques chez le mammifère.

9. Utilisation de β-aléthine selon la revendication 8, dans laquelle la maladie ou le trouble est une maladie ou un trouble auto-immunes, ou une maladie ou trouble immunodéficitaires, y compris le SIDA ou l'hypogammaglobulinémie.

10. Utilisation de β-aléthine selon la revendication 9, comprenant les étapes d'extraction d'un immunocyte du mammifère, d'exposition de l'immunocyte *in vitro* à suffisamment de β-aléthine pour stimuler la fonction ou la bioproduction immunitaires de celui-ci, puis de réintroduction de l'immunocyte stimulé dans le mammifère souffrant.

11. Utilisation de β-aléthine selon la revendication 10, dans laquelle le mammifère souffre de néoplasie, et l'immunocyte est en plus stimulé par exposition à la cellule néoplasique avant la réintroduction de l'immunocyte dans le mammifère.

12. Utilisation de β-aléthine pour la fabrication d'un médicament pour retarder le commencement de la sénescence, comprenant l'exposition d'une cellule présénescente, *in vivo* ou *in vitro*, à de la β-aléthine en une quantité suffisante pour retarder la sénescence de celle-ci.

13. Utilisation de β-aléthine pour la fabrication d'un médicament pour adapter une cellule d'un organisme, résistante à une culture, à la culture, comprenant l'étape d'exposition de la cellule résistante, *in vivo* ou *in vitro*, à de la β-aléthine en une quantité suffisante pour adapter la cellule à la culture.

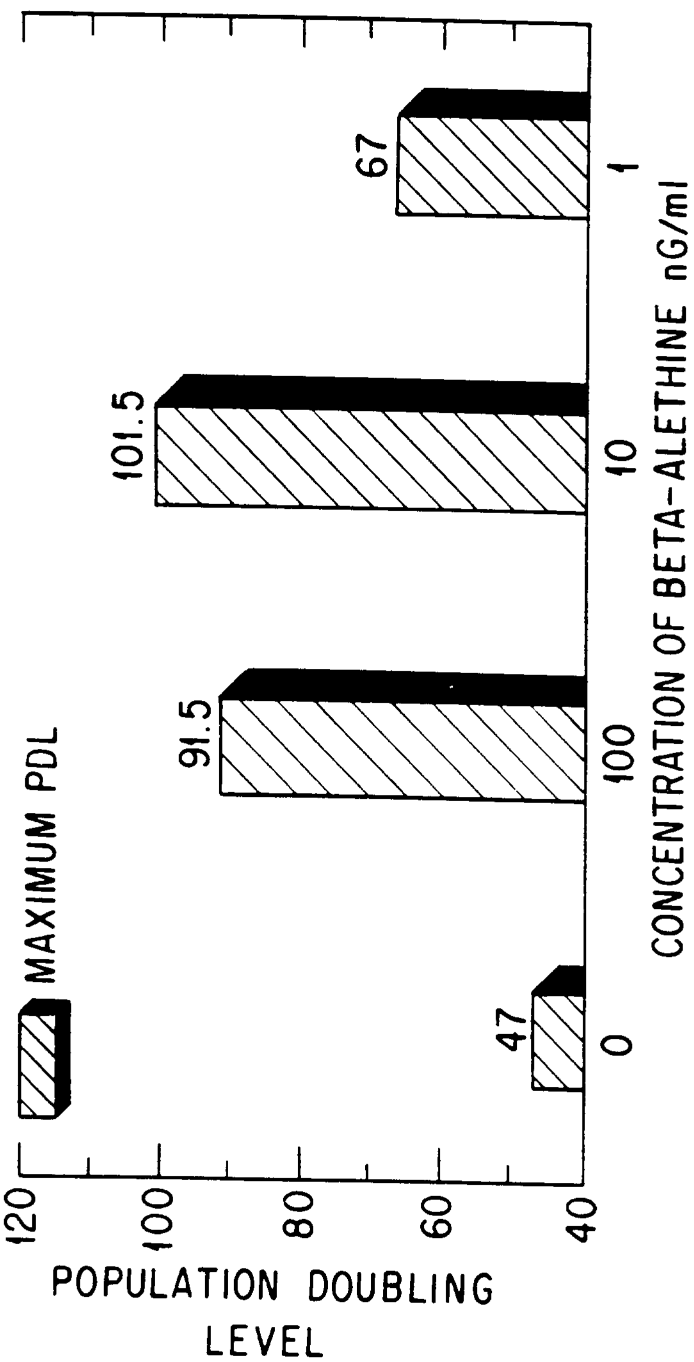
MAXIMUM PDL
POPULATION DOUBLING LEVEL
120
100
80
60
40
47
91.5
101.5
67
0
100
10
1
CONCENTRATION OF BETA-ALETHINE nG/ml

FIG. 1

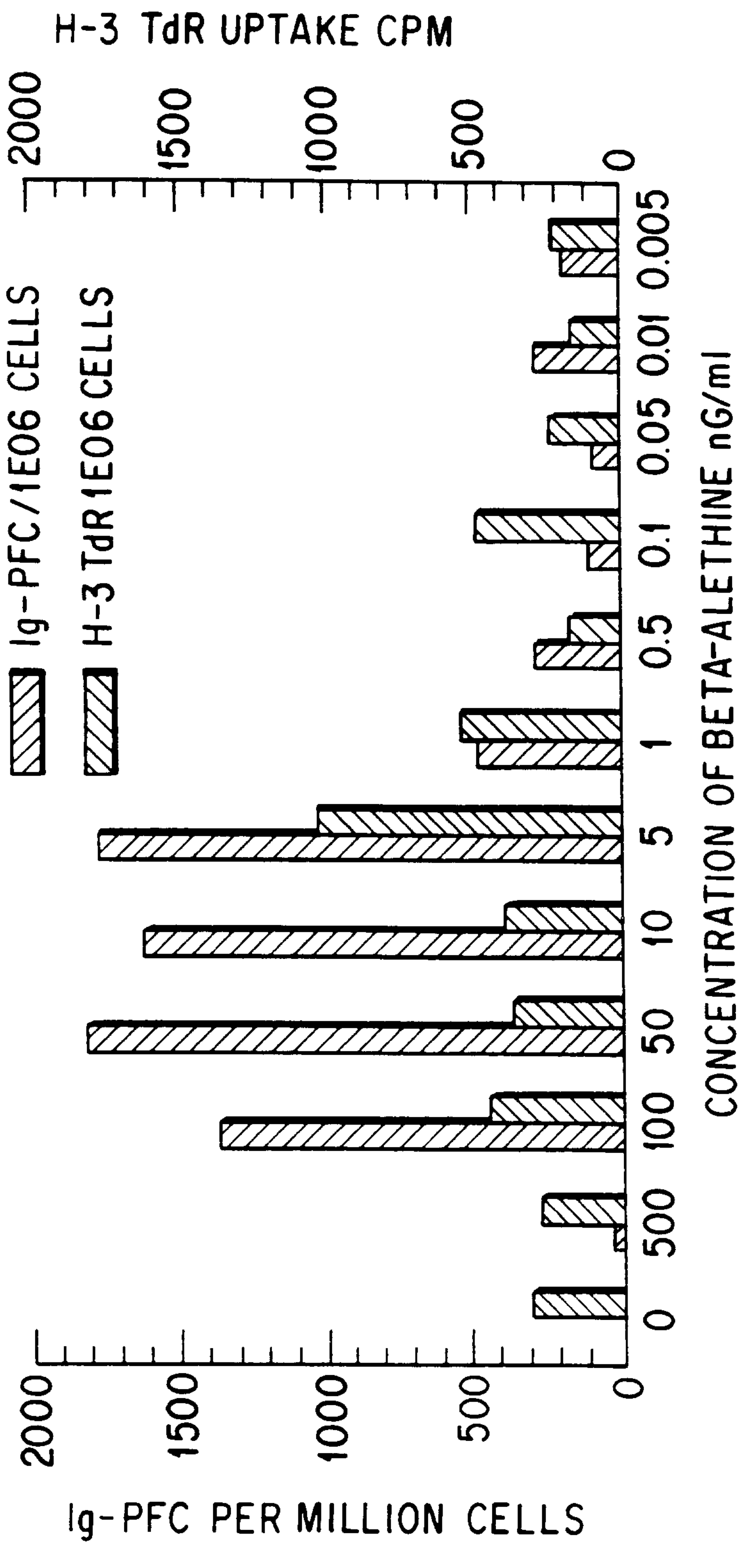
H-3 TdR UPTAKE CPM
2000
1500
1000
500
0
Ig-PFC/1E06 CELLS
H-3 TdR 1E06 CELLS
0.005
0.01
0.05
0.1
0.5
1
5
10
50
100
500
0
CONCENTRATION OF BETA-ALETHINE nG/ml
2000
1500
1000
500
0
Ig-PFC PER MILLION CELLS

FIG. 2

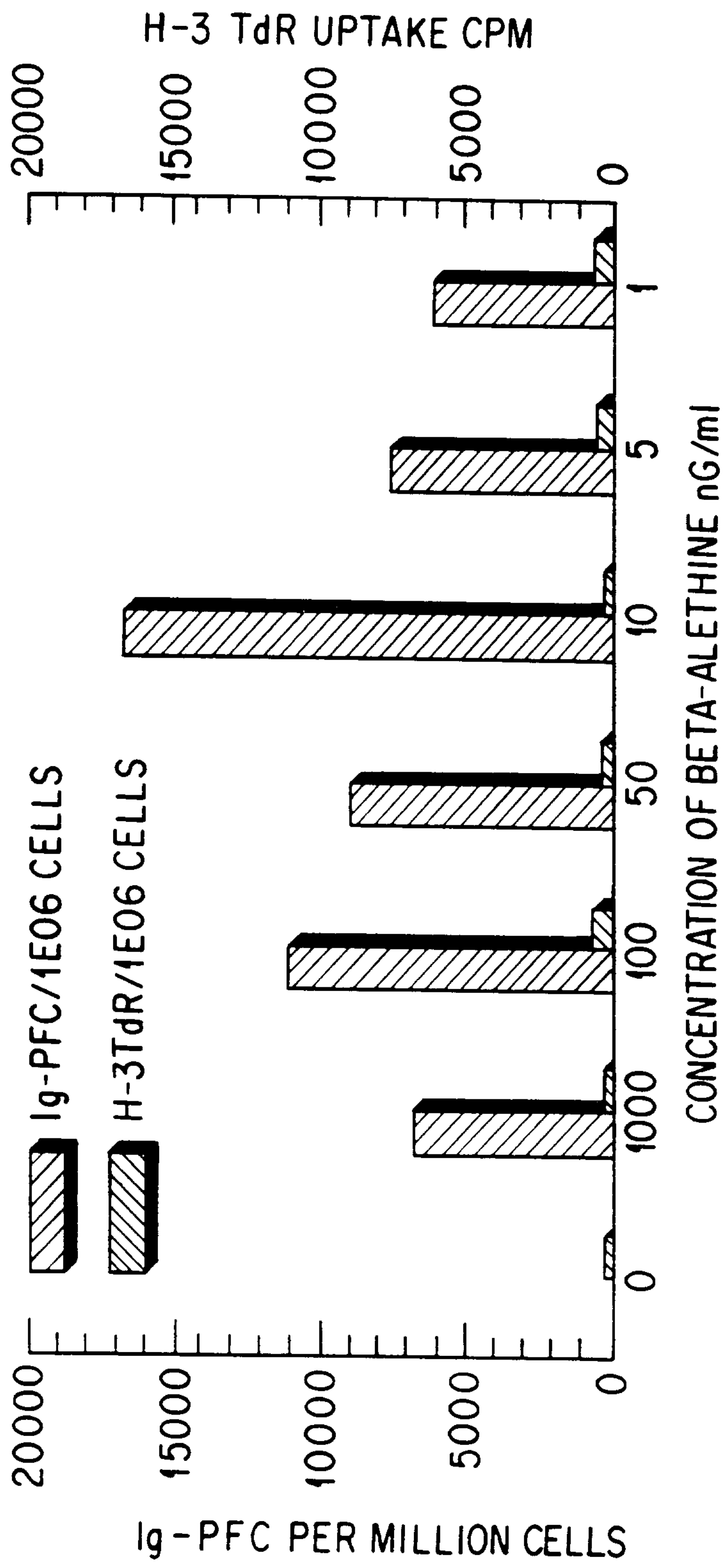
H-3 TdR UPTAKE CPM
20000
15000
10000
5000
0
Ig-PFC/1E06 CELLS
H-3TdR/1E06 CELLS
0
1000
100
50
10
5
1
CONCENTRATION OF BETA-ALETHINE nG/ml
Ig-PFC PER MILLION CELLS

FIG. 3

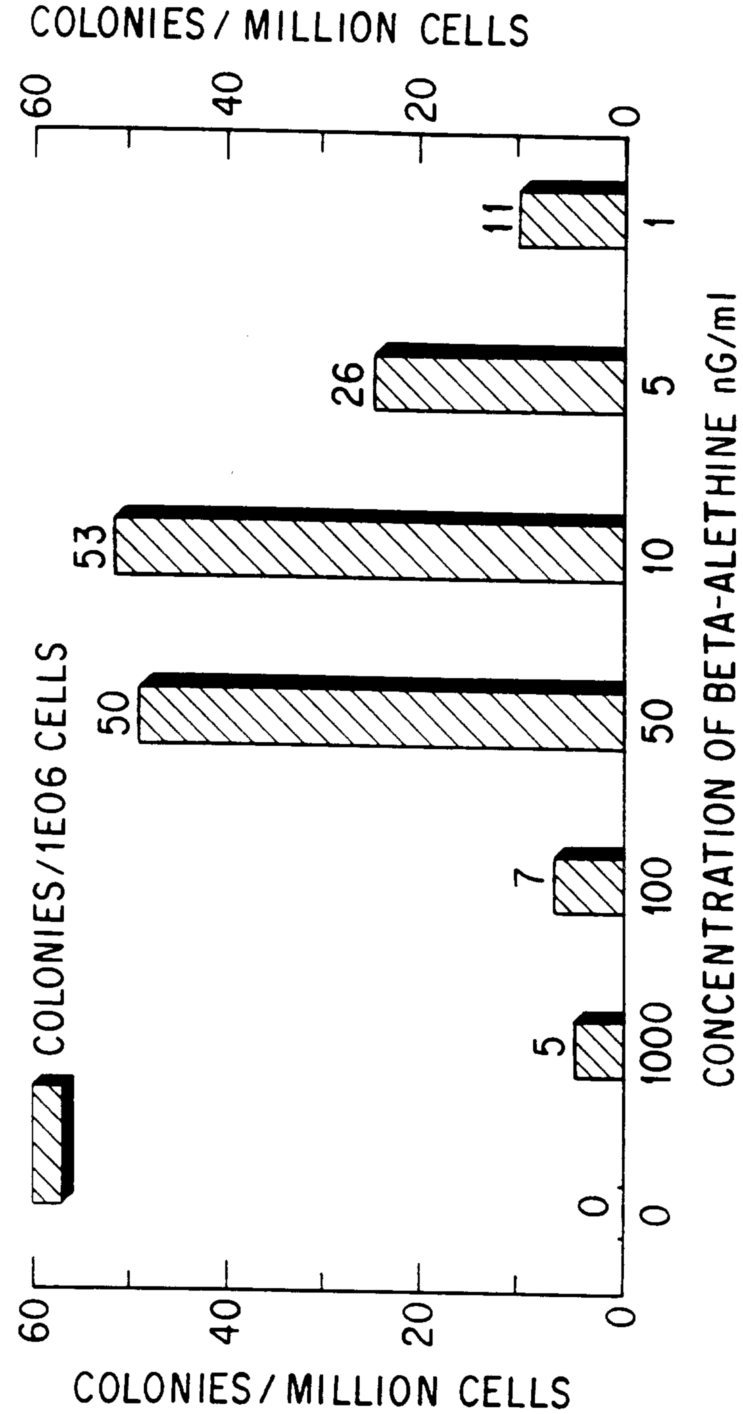
COLONIES/ MILLION CELLS
60
40
20
0
COLONIES/1E06 CELLS
0
5
7
50
53
26
11
0
1000
100
50
10
5
1
CONCENTRATION OF BETA-ALETHINE nG/ml
COLONIES/ MILLION CELLS

FIG. 4

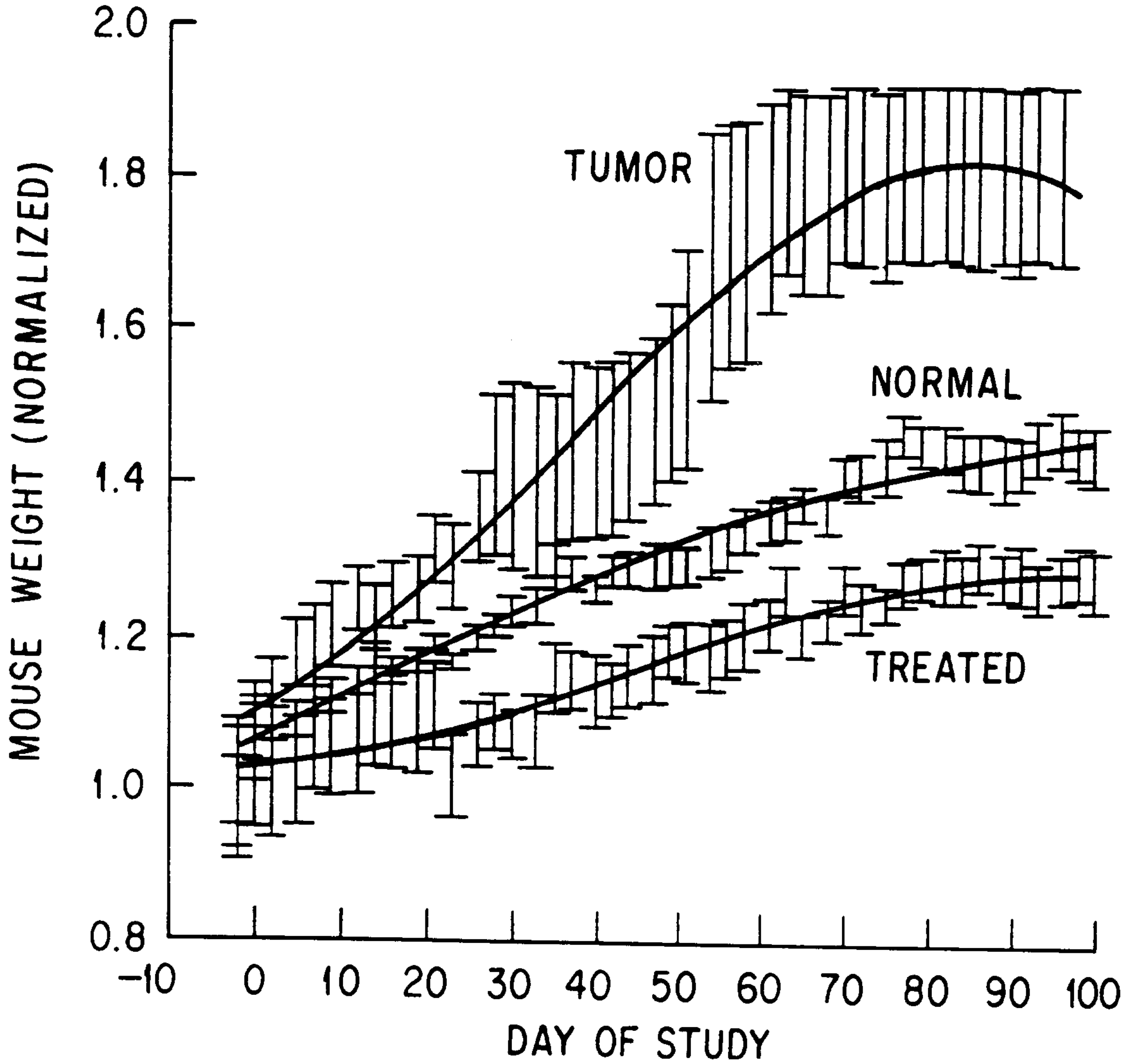
2.0
1.8
1.6
1.4
1.2
1.0
0.8
MOUSE WEIGHT (NORMALIZED)
TUMOR
NORMAL
TREATED
-10
0
10
20
30
40
50
60
70
80
90
100
DAY OF STUDY

FIG. 5

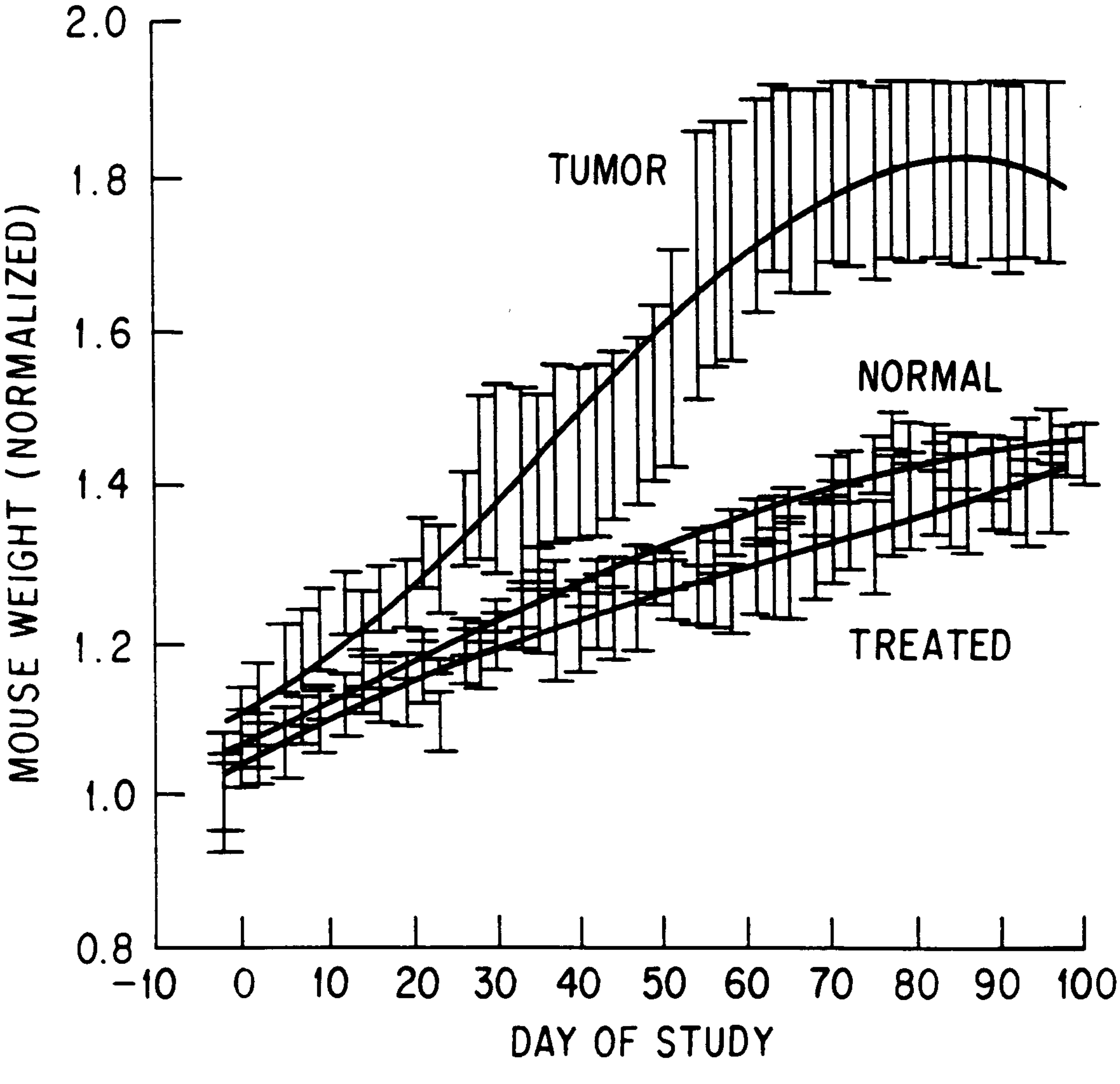
2.0
1.8
1.6
1.4
1.2
1.0
0.8
MOUSE WEIGHT (NORMALIZED)
TUMOR
NORMAL
TREATED
−10
0
10
20
30
40
50
60
70
80
90
100
DAY OF STUDY

FIG. 6

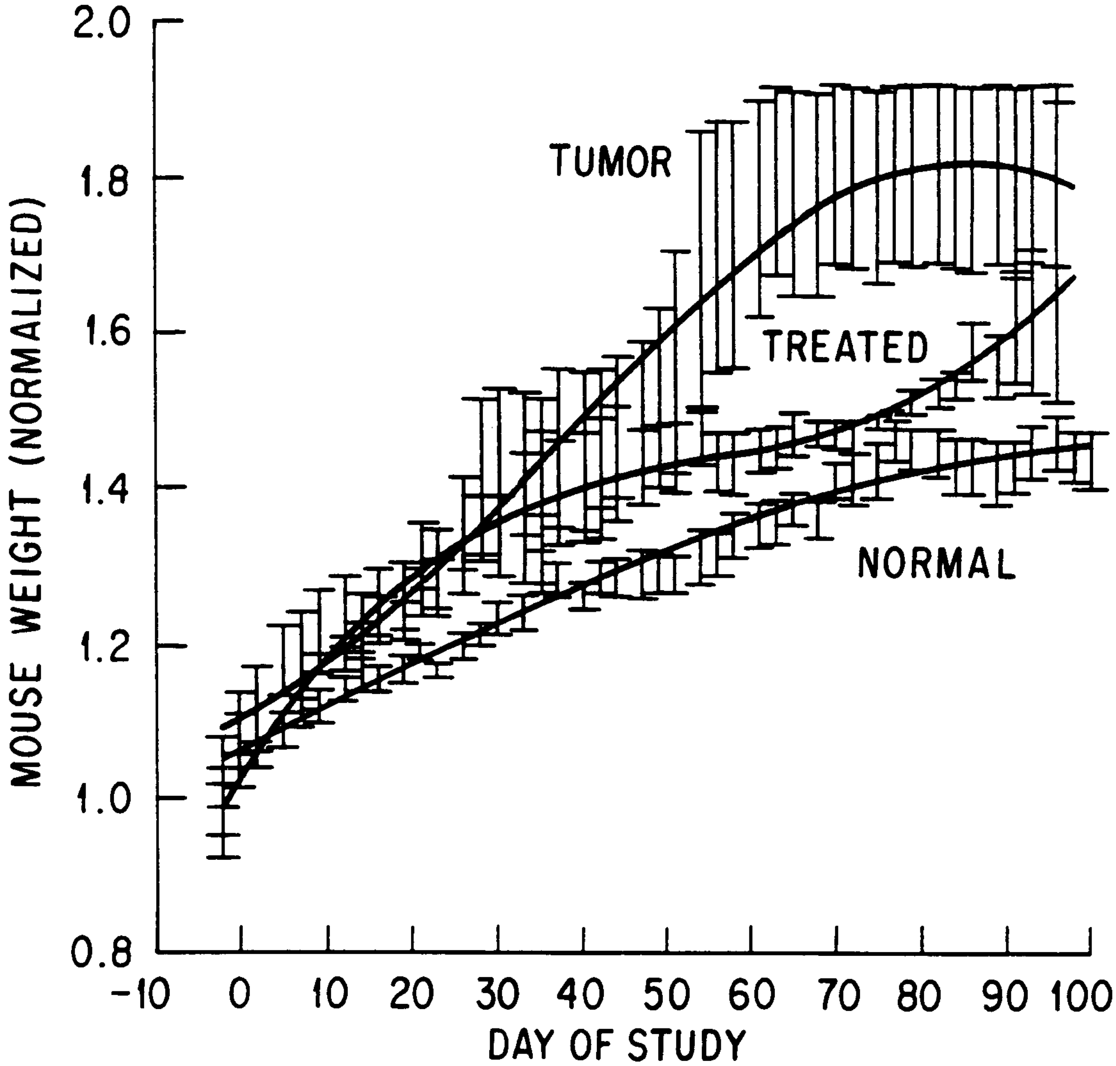
2.0
1.8
1.6
1.4
1.2
1.0
0.8
MOUSE WEIGHT (NORMALIZED)
TUMOR
TREATED
NORMAL
-10
0
10
20
30
40
50
60
70
80
90
100
DAY OF STUDY

FIG.7

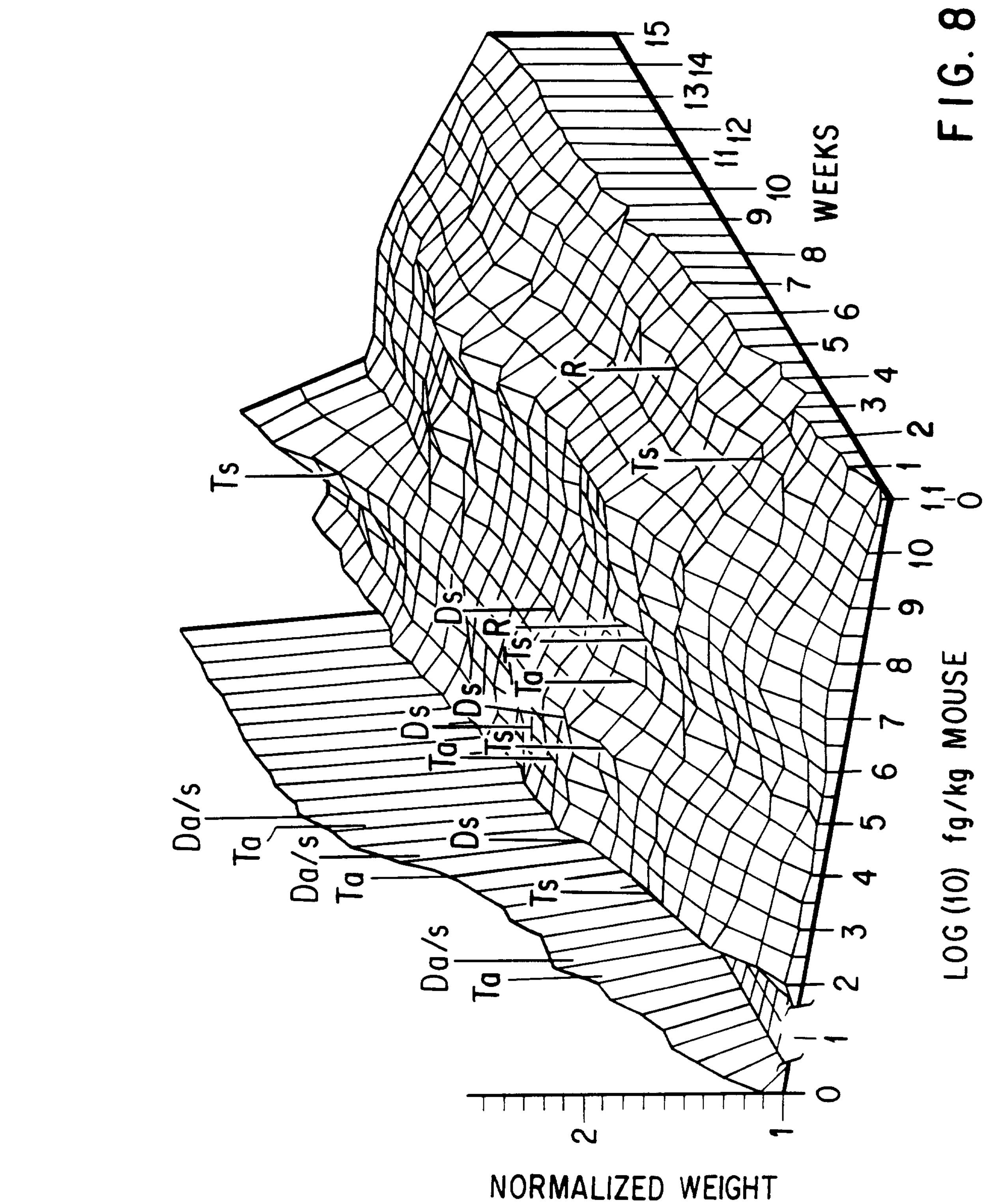
Ts
Da/s
Ta
Da/s
Ta
Da/s
Ta
Ds
Ta
Ds
Ds
Ts
Ta
Ts
R
Ds
Ts
R
Ts
WEEKS
LOG (10) fg/kg MOUSE
NORMALIZED WEIGHT
2
1

FIG. 8

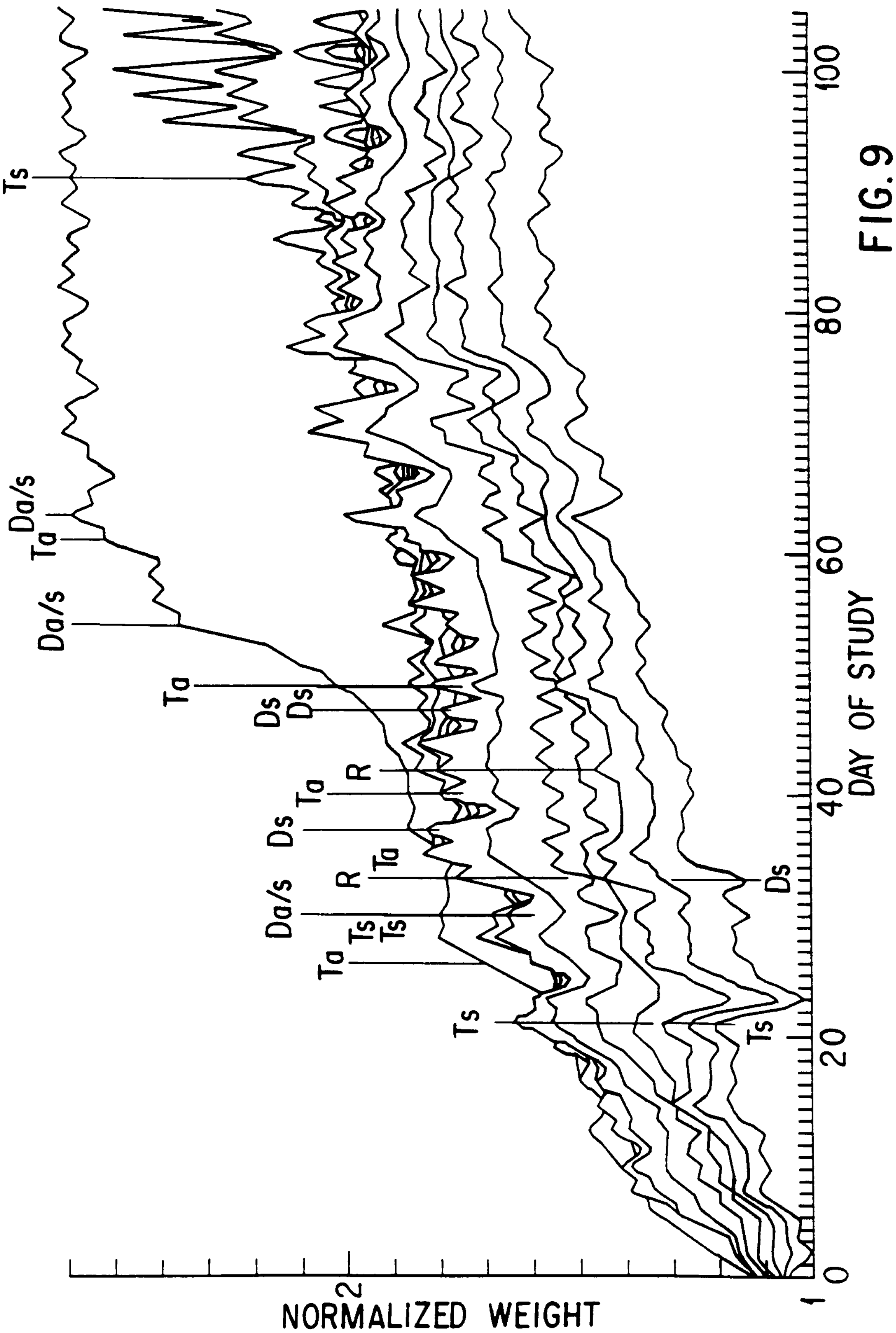
NORMALIZED WEIGHT
1
2
DAY OF STUDY
0
20
40
60
80
100
Ts
Ta
Ts
Ts
Da/s
R
Ta
Ds
Ta
R
Ds
Ds
Ta
Da/s
Ta
Da/s
Ts
Ts
Ds

FIG.9

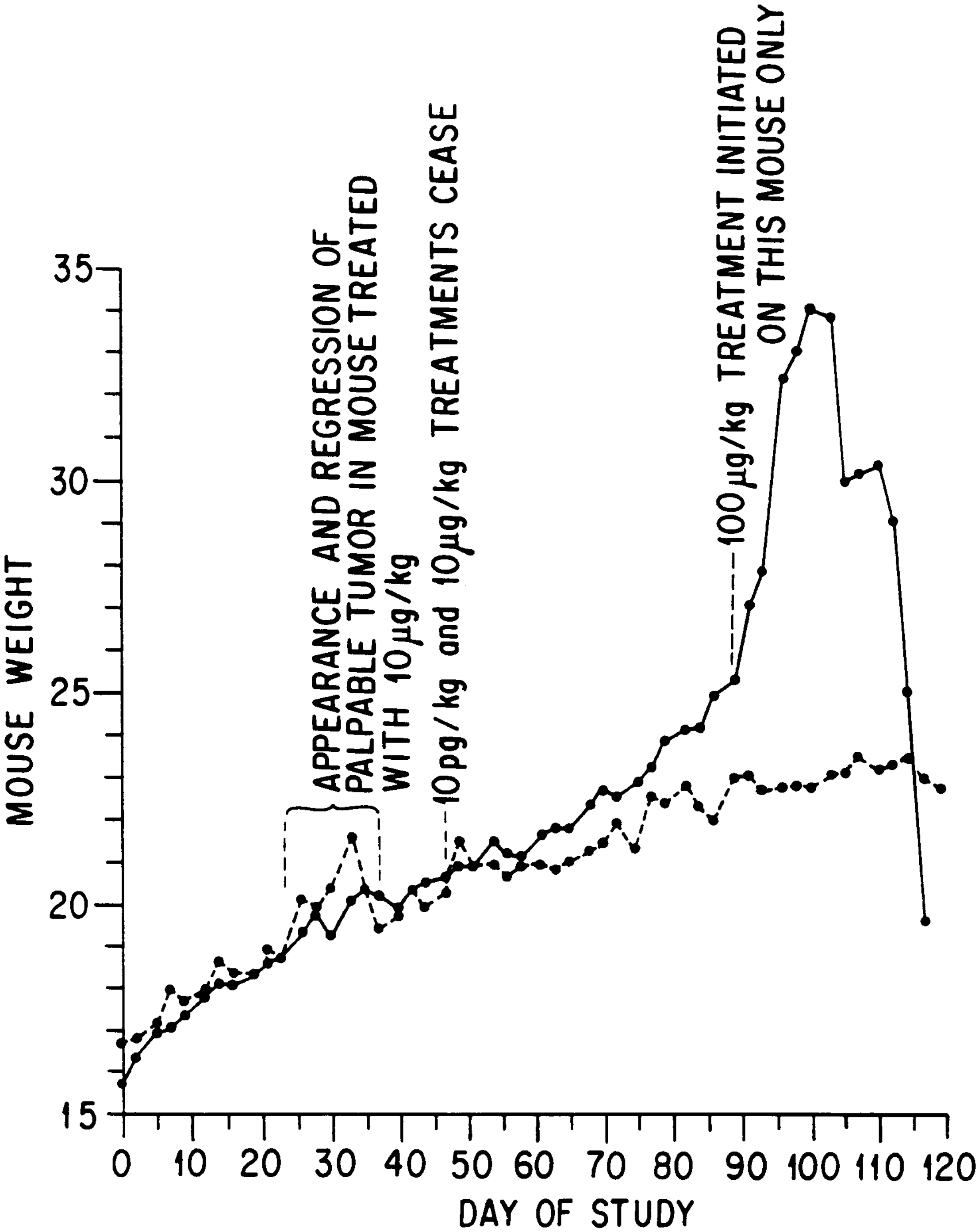

MOUSE WEIGHT
35
30
25
20
15
0 10 20 30 40 50 60 70 80 90 100 110 120
DAY OF STUDY
APPEARANCE AND REGRESSION OF PALPABLE TUMOR IN MOUSE TREATED WITH 10μg/kg
10pg/kg and 10μg/kg TREATMENTS CEASE
100μg/kg TREATMENT INITIATED ON THIS MOUSE ONLY


FIG. 10